# EUROPEAN PATENT SPECIFICATION

(11) **EP 2 001 899 B1**
(45) Date of publication and mention of the grant of the patent: **08.10.2014**
(21) Application number: 07711948.5
(22) Date of filing: 09.03.2007
(51) Int. Cl.: G01N 33/68, C07K 7/06, C07K 17/00, C07K 14/05, C07K 14/705, C07K 7/64

(54) **PEPTIDES AGAINST AUTOANTIBODIES ASSOCIATED WITH GLAUCOMA AND USE OF THESE PEPTIDES**
PEPTIDE GEGEN MIT GLAUKOM ASSOZIIERTEN AUTOANTIKÖRPERN, UND VERWENDUNG DIESER PEPTIDE
PEPTIDES DIRIGÉS CONTRE DES AUTO-ANTICORPS ASSOCIÉS AU GLAUCOME ET UTILISATION DE CES PEPTIDES

(30) Priority: 09.03.2006 EP 06075594; 09.03.2006 US 780761 P
(43) Date of publication of application: 17.12.2008
(73) Proprietor: Max-Delbrück-Centrum für Molekulare Medizin (MDC), 13125 Berlin (DE); Fresenius Medical Care Deutschland GmbH, 61346 Bad Homburg (DE)
(72) Inventor: WALLUKAT, Gerd, 13129 Berlin (DE); KUNZE, Rudolf, 13187 Berlin (DE); HERRMANN, Martin, 91077 Neunkirchen (DE); VOLL, Reinhard, 91330 Eggolsheim (DE); JÜNEMANN, Anselm, 91054 Erlangen (DE); SCHLÖTZER, Ursula, 91054 Erlangen (DE)
(74) Representative: Boeckh, Tobias
(86) International application number: PCT/EP2007/002269
(87) International publication number: WO 2007/101732

(56) References cited:
- WO-A-01/21660
- WO-A-99/62933
- WO-A-2004/036220
- WO-A1-97/17980
- MAGNUSSON Y ET AL: "Antigenic analysis of the second extra-cellular loop of the human beta-adrenergic receptors." CLINICAL AND EXPERIMENTAL IMMUNOLOGY. OCT 1989, vol. 78, no. 1, October 1989 (1989-10), pages 42-48, XP009070741 ISSN: 0009-9104
- GUILLET J G ET AL: "Induction of a pharmacologically active clonotypic B cell response directed to an immunogenic region of the human beta 2-adrenergic receptor." CLINICAL AND EXPERIMENTAL IMMUNOLOGY. SEP 1992, vol. 89, no. 3, September 1992 (1992-09), pages 461-467, XP009070742 ISSN: 0009-9104
- BANKS W A ET AL: "ENTRY OF DELTA SLEEP-INDUCING PEPTIDES INTO DOG CEREBROSPINAL FLUID ROLE OF PHYSICOCHEMICAL AND PHARMACOKINETIC PARAMETERS" BRAIN RESEARCH BULLETIN, vol. 17, no. 2, 1986, pages 155-158, XP009070766 ISSN: 0361-9230
- GRUS FRANZ H ET AL: "Complex autoantibody repertoires in patients with glaucoma." MOLECULAR VISION [ELECTRONIC RESOURCE]. 25 FEB 2004, vol. 10, 25 February 2004 (2004-02-25), pages 132-137, XP009070754 ISSN: 1090-0535
- MACH R. ET AL: "[Steroid glaucoma in severe atopic keratoconjunctivitis. A reminder for possible therapy with plasmapheresis]", CESKOSLOVENSKÁ OFTALMOLOGIE MAY 1992 LNKD- PUBMED:1525888, vol. 48, no. 3, May 1992 (1992-05), pages 167-170, ISSN: 0009-059X
- RÖNSPECK W ET AL: "Peptide based adsorbers for therapeutic immunoadsorption", THERAPEUTIC APHERESIS AND DIALYSIS, BLACKWELL PUBLISHING ASIA, CARLTON SOUTH, AU, vol. 7, no. 1, 1 February 2003 (2003-02-01), pages 91-97, XP002313965, ISSN: 1744-9979, DOI: 10.1046/J.1526-0968.2003.00017.X

## Description

The invention relates to a peptide according to claim 1, the use of said peptide as a medical substance and the use of said peptide in the diagnosis or therapy of glaucoma.

A glaucoma, preferred a primary open angle glaucoma, an ocular hypertension, a normal tension glaucoma, an acute angle-closure glaucoma, a primary congenital glaucoma, a pseudoexfoliant glaucoma and/or a secondary glaucoma surprisingly are often accompanied by the prevalence of antibodies directed against beta-2-adrenergic receptor. These antibodies can act as agonistic autoantibodies; these autoantibodies activate for example the beta-2-adrenergic receptor or the beta-adrenergic system.

The invention relates more specifically to nudeic acid molecules encoding peptides which interact with autoantibodies associated with glaucoma, to the peptides themselves, to a pharmaceutical composition comprising said nudeic acid molecules and peptides, and to the use of said peptides - especially in apheresis - for the treatment of glaucoma.

Polycellular organisms, especially mammals, and more particularly humans, cope with their environment via various biochemical and/or immunological regulatory mechanisms. For example, an important bio-chemical regulatory mechanism is the one that is responsible for maintaining constant a specific internal temperature in the organism. Another important regulatory mechanism provides cellular and humoral substances, such as antibodies, enabling the organism to cope successfully with microorganisms, parasites, but also, tumors or other diseases. Above all, the function of antibodies is based on the capability of distinguishing between "foreign" and "self". That is, the organism itself produces antibodies which are designed in such a way that endogenous structures such as tissues or organs are not attacked, but bind to bacteria, parasites or other exogenic components, thus initiating or supporting immunological reactions.

During biochemical development of an antibody response in the organism, somatic hypermutations of antibody-producing B cells occur during T cell-dependent immune response. Such mutations selectively involve immunoglobulin genes, being essential to the formation of precursor cells of antibody-forming cells with high affinity. It should be noted in this context that such mutation is a normal and essentially non-pathogenic process. However, mutations may also give rise to the formation of highly affine autoantibodies, thus doing damage to the organism.

Autoantibodies are closely associated with the development of autoimmune diseases or causally responsible for such diseases. Antibody-mediated autoimmune diseases also include diseases such as rheumatism or lupus erythematosus. Numerous autoimmune diseases, being highly inconvenient to affected persons, relate to important biochemical cycles in the organism.

It was not known to date that glaucoma is a group of diseases which is associated with autoantibodies. In the controversial state of the art, autoantibodies or antibodies were described by certain authors as a possible diagnostic means for eye diseases (WO 2004/036220 and Grus, Franz H. et al., February 2004, Molecular Vision). It was however utterly surprising that ligands for autoantibodies may be applied in the therapy of Glaucoma. Glaucoma represents a group of destruction of the optic nerve involving loss of retinal ganglion cells in a characteristic pattern of optic neuropathy. Although raised intraocular pressure is a significant risk factor for developing glaucoma, there is no set threshold for intraocular pressure that causes glaucoma. One person may develop nerve damage at a relatively low pressure, while another person may have high eye pressures for years and yet never develop damage. Untreated glaucoma leads to permanent damage of the optic nerve and resultant visual field loss, which can progress to blindness.

While glaucoma mayor may not have distinct symptoms, an almost inevitable complication of glaucoma is vision loss. Visual loss from glaucoma first affects peripheral vision. Early vision loss is subtle, and is not noticed by the patient. Moderate to severe vision loss may be noticed by the patient by checking his peripheral vision thoroughly. This can be done by dosing one eye and examining all four corners of the visual field for clarity and sharpness, then repeating with the other eye dosed. All too often, the patient does not notice the loss of vision until he experiences "tunnel vision". If the disease is not treated, the visual field will become more and more narrow, obscuring central vision, and finally progressing to blindness in the affected eye(s).

Waiting for symptoms of visual loss to occur is not optimal care. Visual loss related to glaucoma is irreversible, but can be prevented or slowed by treatment. An optometrist or ophthalmologist should be consulted by people at risk for glaucoma.

Although intraocular pressure is only one of the causes of glaucoma, lowering it via pharmaceuticals or surgery is currently the mainstay of glaucoma treatment.
Intraocular pressure can be lowered with medication, usually eye drops. There are several different classes of medications to treat glaucoma with several different medications in each class. Topical beta-adrenergic receptor antagonists such as timolol, levobunolol, and betaxold decrease aqueous humor production by the ciliary body. Alpha2-adrenergic agonists such as brimonidine (Alphagan) work by a dual mechanism, decreasing aqueous production and increasing uveo-scleral outflow. Less-selective sympathomimetics like epinephrine and dipivefin (Propine) increase outflow of aqueous humor through trabecular meshwork and possibly through uveoscleral outflow pathway, probably by a beta2-agonist action. Miotic agents (parasympathomimetics) like pilocarpine work by contraction of the ciliary muscle, tightening the trabecular meshwork and allowing increased outflow of aqueous through traditional pathways. Carbonic anhydrase inhibitors like dorzolamide (Trusopt), brinzolamide (Azopt), acetazolamide (Diamox) lower secretion of aqueous humor by inhibiting carbonic anhydrase in the ciliary body. Prostaglandin analogs like latanoprost (Xalatan), bimatoprost (Lumigan) and travoprost (Travatan) increase uveoscleral outflow of aqueous.

Marijuana has been shown to lower the intraocular pressure in some eyes in a few studies but this is generally not used clinically.

The possible neuroprotective effects of various tropical and systemic medications are also being investigated.

Both laser and conventional surgeries are performed to treat glaucoma. Laser trabeculoplasty may be used to treat open angle glaucoma. An argon or Nd:YAG laser spot is aimed at the trabecular meshwork to stimulate opening of the mesh to allow more outflow of aqueous fluid. Laser peripheral iridectomy may be used in patients susceptible to angle closure glaucoma. In it, the laser is aimed at the iris to make an opening in it. This allows a new channel for fluid to flow when the usual channel through the dilated pupil is blocked.

The most common conventional surgery performed for glaucoma is the trabeculectomy. Here, a partial thickness flap is made in the scleral wall of the eye, and a window opening made under the flap to remove a portion of the trabecular meshwork. The scleral flap is then sutured loosely back in place. This allows fluid to flow out of the eye through this opening, resulting in lowered intraocular pressure.

There are also several different small tubes that are inserted into the anterior chamber of the eye and out underneath the conjunctiva to allow flow of fluid out of the eye.

Disadvantageously, however, routine use of this new therapeutical method has not been possible for capacity reasons. The known drops have bad compliance.

The object of the invention was therefore to provide compounds allowing easy, reliable and effective diagnosis and therapy of glaucoma.

The invention solves the above technical problem by providing a peptide consisting of an amino acid sequence X1-X2-X3-X4-X5-X6-X7-X8-X9 wherein
X1= amino group, amide, acetyl group, biotin group, marker, spacer, linker, GKK, SGKK or deletion,
X9 = amino group, amide, acetyl group, biotin group, marker, spacer, linker, GKK, SGKK or deletion, wherein
X2 to X8 of the amino acid sequence X1-X2-X3-X4-X5-X6-X7-X8-X9 is selected from a group that consists of
   (i) a peptide consisting of the amino acid sequence GLQSWGQ, SVEATSE, VADMSVD, AQNTCCN, GNQSSSQ, SEEYAHE, AINCYAN and/or ANETCCD;
   (ii) a peptide consisting of an amino acid sequence having a sequence with 90% homology to an amino acid sequence in accordance with (i);
   (iii) a peptide according to amino acid sequence (i) or (ii) which is modified by branch or extension with the same or another peptide according to amino acid sequence (i) or (ii) to form a homooligomeric or heterooligomeric peptide.

In another aspect, the invention provides an isolated nudeic acid molecule - and a peptide encoded by same - selected from the group comprising:
a) a nudeic acid moleculeconsisting of a nudeotide sequence which encodes at least one peptide selected from the group consisting of peptides according to daim 1, preferred GLQSWGQ, SVEATSE, VADMSVD, AQNTCCN, GNQSSSQ, SEEYAHE, more preferred AINCYAN and/or ANETCCD;
b) a nudeic acid molecule which is complementary to a nudeotide sequence in accordance with a);
c) a nudeic acid molecule which undergoes hybridization with a nucleotide sequence according to a) or b) under stringent conditions;
d) a nudeic acid molecule consisting of a nudeotide sequence having sequence with 90% homology to a nudeotide sequence according to a), b) or c);
e) a nudeic acid molecule which, as a consequence of the genetic code, is degenerated into a nucleotide sequence according to a) through d).

Accordingly, the invention involves the surprising teaching that the nudeic acid molecules according to the invention, as well as the peptides encoded by same, can be used for diagnosis and therapy of diseases of the optic nerve. More specifically, the biological structures, preferably peptides, encoded by said nudeic acid molecules can be used in various forms in the prophylaxis, diagnosis, therapy, follow-up and/or aftercare of diseases of the optic nerve, especially involving loss of retinal ganglion cells, preferably glaucoma.

In a preferred embodiment of the invention the nudeic acid molecule which has sufficient homology to be functionally analogous to the nudeic acid sequence in accordance with a), b) and/or c) has at least 90% homology to a nudeotide sequence according to a), b) or c). In the meaning of the invention the term "to be functionally analogous to the above-mentioned nudeotide sequences or to the sequences hybridizing with said nudeotide sequences" means that the homologues exhibit a behavior in diseases of the optic nerve, especially glaucoma, such that reliable and effective use thereof is possible in diagnosis and/or therapy of such diseases or pathogenic conditions associated with said diseases. Functionally analogous sequences in the meaning of the invention are all those sequences which can be identified as equally effective by a person skilled in the art, using routine tests.

In another advantageous embodiment of the invention the nudeic acid molecule has at least 90% homology to the nudeic acid molecule in accordance with a), b) or c).

In another preferred embodiment of the invention the nudeic acid molecule is a genomic DNA, a cDNA and/or an RNA.

The invention also relates to a vector comprising a nudeic acid molecule according to the invention and, in addition, to a host cell comprising said vector.

In a preferred fashion the invention also relates to a peptide encoded by a nudeic acid molecule according to the invention and by the preferred functionally analogous nudeic acid molecules. Surprisingly, the peptide according to the invention can be used in the diagnosis and/or therapy of diseases associated with diseases of the optic nerve. More specifically, the peptides of the invention are bound by autoantibodies in patients suffering from glaucoma.

That is, the invention relates to all peptides encoded by the nudeic acid molecules according to the invention, preferably those having at least 90% homology in accordance with d). Obviously, said functionally analogous nudeic acid molecules can be modified by deletion, addition, substitution, translocation, inversion and/or insertion in such a way that the peptides encoded by same interact with autoantibodies associated with the glaucoma. By virtue of the teaching according to the invention, a person skilled in the art will be capable of generating other equivalent peptides functionally analogous to peptides having the sequence of daim 1, preferred AINCYANETCCD, AIDCYAKETCCD, GLQSWGQ, SVEATSE, VADMSVD, AQNTCCN, GNQSSSQ, SEEYAHE, more preferred AINCYAN and/or ANETCCD, respectively. Of course, it is also possible to use peptides comprising the above-mentioned structures. Obviously, however, the peptides (as a substance, molecule or a mixture of substances) according to the invention would not be selected in a way so as to completely represent the naturally occurring human beta-2-adrenergic receptor. With reference to the methods of the invention and the uses according to the invention, it can be advantageous to use the human beta-2-adrenergic receptor or parts thereof as specific ligands for removing significant portions of the immunoglobulin from plasma taken from a patient, suffering from glaucoma. This means that the substance claims refer to specific parts of the receptor (the peptides) and the use or method claims refer to the use of the whole receptor for the treatment of glaucoma. A person skilled in the art knows the definition of a ligand according to the invention. Ligands or specific ligands in the context of the invention are means which interact specifically with autoantibodies directed against the beta-2-adrenergic receptor, most preferred against the second extracellular loop of said receptor. In another preferred embodiment of the invention, the ligands or specific ligands consist of the peptides of the invention. The peptides according to the invention feature advantages in comparison to those known in the state of the art (Magnusson, Y. et al., Clin. exp.Immunol, 1989; Guillet, J.-G. et al., Clin. exp. Immunol., 1992; Banks, W. A et al., Brain Research Bulletin, 1986; WO 2004/036220; Grus, Franz H. et al., Molecular Vision, 2004; WO 01/21660; WO 99/62933). The peptides of the invention show a better interaction with the autoantibodies than the peptides known in the state of the art. Furthermore, the peptides of the invention bind better to the matrix of a column and when binding to a column, they lose less of their antibody-binding activity than the peptides of the state of the art. The autoantibodies associated with glaucoma can be bound very well with the peptides of the invention. If a less specific binding is necessary, the invention discloses the technical teaching for extracting the antibodies or autoantibodies from the plasma or blood of a human (for example by using an unspecific column) and for consequently reinfusing the needed antibodies to the patient, needed antibodies being such which are important for the immune system of a patient. The documents of the state of the art do not indicate that the peptides disclosed therein may be used for generating antibodies, especially agonistic antibodies which can be used for the treatment of glaucoma.

In another preferred embodiment, the invention relates to the use of a specific ligand for human immunoglobulin selected from the peptides according to daim 1 in the manufacture of a column having said ligand coupled thereto for the treatment of a patient suffering from glaucoma, said treatment comprising passing plasma of the patient over the column under conditions which effect the binding of said specific ligand to immunoglobulin in the patient's plasma, thereby removing a significant portion of the immuno-globulin from the patient's plasma and reinfusing the plasma to the patient In the context of the invention, the term "significant portion" is directed to an amount of autoantibodies whose removal from the plasma leads to an improvement of the patient's condition or to the non-appearance of a further aggravation. This improvement of the patient's condition relates to at least one of the aspects of glaucoma, known to a person skilled in the art. According to the invention, the specific ligand is the peptides according to the invention. These aspects of the invention are based on the conclusion made by the inventors, that glaucoma is caused by autoantibodies, especially by agonistic autoantibodies.

The invention also encompasses the use of a peptide according to daim 1 in the manufacture of a column for extracorporal removal of autoantibodies directed against glaucoma structures by removing immunoglobulins of any or all classes and subclasses, for the treatment of glaucoma. The term "glaucoma structures" is known to a person skilled in the art and refers to structures associated with this disease (In the meaning of PCT/US96/18457, where such a structure is disclosed in the context of cardiomyopathy as "cardiac structure".). The term "glaucoma structure" therefore refers to any structure of the eye or the optic nerve which is modified by glaucoma or whose modification causes glaucoma. A "glaucoma structure" is a binding site of said autoantibody, especially of agonistic autoantibodies.

Another aspect of the invention is therefore also the removal of immunoglobulins from plasma taken from a patient suffering from glaucoma using the peptides of daim 1 and reinfusing said plasma to the patient. The reinfused plasma may also contain those immunoglobulins which induce a positive effect in the context of the patient's immune system.

The invention also encompasses the use of a peptide according to daim 1 in the manufacture of a column for extracorporal removal of autoantibodies which are directed against the second extracellular loop of the beta-2-adrenergic receptor. This means that the removal of autoantibodies directed against the second extracellular loop of the beta-2-adrenergic receptor is part of the invention.

The peptides according to the invention relate to selected regions of the second extracellular loop of the beta-2-adrenergic receptor. However, the advantageous sequences X1-X2-X3-X4-X5-X6-X7-X8-X9 wherein
X1 = amino group, amide, acetyl group, biotin group, marker, spacer, linker, GKK, SGKK or deletion,
X9 = amino group, amide, acetyl group, biotin group, marker, spacer, linker, GKK, SGKK or deletion, wherein
X2 to X8 of the amino acid sequence X1-X2-X3-X4-X5-X6-X7-X8-X9 is selected from a group that consists of
   (i) a peptide consisting of the amino acid sequence GLQSWGQ, SVEATSE, VADMSVD, AQNTCCN, GNQSSSQ, SEEYAHE, AINCYAN and/or ANETCCD;
   (ii) a peptide consisting of an amino acid sequence having a sequence with 90% homology to an amino acid sequence in accordance with (i);
   (iii) a peptide according to amino acid sequence (i) or (ii) which is modified by branch or extension with the same or another peptide according to amino acid sequence (i) or (ii) to form a homooligomeric or heterooligomeric peptide.
may also comprise further amino acids naturally flanking the above-mentioned sequences in the second extracellular loop of the receptor, in which case the size of the flanking regions should not be selected such that the receptors themselves would be selected. Examples of such sequences - expanded by flanking or other artificial regions - are HWYRATHQE-AINCYAN - a peptide of the invention with a sequence of the extracellular loop flanking N-terminal region and ANETCCD-FFTNQ a peptide of the invention with a loop sequence flanking C-terminal region. In these cases the flanking sequences are representing a linker or spacer of a part thereof.

Consequently, the peptides in the meaning of the invention can be designed in such a way and may include such a number of additional amino acids, spacers or other structures that they are suitable for interaction with the antibodies, and preferably in such a way that they represent an epitope for the latter.

Accordingly, the peptides according to the invention are not restricted to the sequences AINCYAN and/or ANETCCD, but rather, the peptides are preferably antibody epitopes essentially including the above-mentioned or functionally analogous sequences, so that they represent and characterize the epitope in such a way that the antibodies would undergo specific interaction with them. Therefore, under particular conditions the terms "epitope" and "peptide" may also be used synonymously.

Furthermore, it is possible to replace single amino acids or groups of amino acids without adversely affecting the activity of the peptides with respect to accomplishing the object of the present invention. For replacement of such amino acids, reference is made to appropriate standard textbooks of biochemistry and genetics. Various ways of preparing peptides have been disclosed in the prior art Peptides designed starting from the peptides of the invention using such methods are included in the teaching according to the invention. For example, one way of generating functionally analogous peptides has been described in PNAS USA 1998, Oct. 13, 95, 12179-84; WO 99/6293 and/or WO 02/38592.

A second way of generating peptide sequences starting from the peptides of invention is the subtitutional analysis (Mol. Diversity 2004, 8, 281-290; J. of Immunol. Methods 2002, 267, 37-51 The substitutional analysis is carried out by systematical replacement of each amino acid by all amino acids/building blocks of your interest. The activity of these peptides/constructs shows the ability of those substitutions. The resulting sequences could have improved properties i.e. higher stability and/or lower hydrophicity).

Another method to generate peptide structure anologues is the synthesis of retro-inverso peptides. Using this method the L-amino acids of the peptides of invention were substituted by D-amino acids with simultaneously inversion of the direction of the peptide bounds. This method is published e.g. in Biochemistry 2001, Apr. 19, 40, 5720-27; Biopolymers (Peptide Science) 2005, Feb. 23, 80, 67-84. The generated peptides are structurally analogue to the peptides of the invention. But due to the use of D-amino acids, these peptides are stable against proteolytic degradation. One example of such peptide sequence is the sequence: naycniG. This sequence is the retro-inverso sequence of a peptide of the invention, in which several amino acids were replaced by other amino acids with similar properties. The small letters indicate D-amino acids. Glycine (G) doesn't exist in two different D- or L-structures.

That is, all peptides, peptide fragments or structures comprising peptides generated using the methods mentioned above - starting from the peptides of the invention - are peptides according to the invention, provided they accomplish the object of the invention and, in particular, interact with the pathogenic autoantibodies. For example, these autoantibodies - via dimerization - can be agonistic autoantibodies activating receptors. Some preferred receptors will be described below.

For example, the peptide sequences can be a natural component of the second extracellular loop of the human beta-2-adrenergic receptor. Surprisingly, the autoantibodies specifically interact with the second extracellular loop or with the loop of the respective beta-2-adrenergic receptor. Accordingly, the invention also relates to the surprising teaching that the major epitope of the autoantibodies associated with diseases of the optic nerve, especially glaucoma and especially preferably primary open angle glaucoma (POAG), are peptides having the amino acid sequences of daim 1, preferred GLQSWGQ, SVEATSE, VADMSVD, AQNTCCN, GNQSSSQ, SEEYAHE, more preferred AINCYAN and/or ANETCCD. Preferably, this sequence is entirely identical on beta-2-adrenergic receptor. Accordingly, the autoantibodies can be antibodies acting as agonistic autoantibodies, i.e., these autoantibodies are capable of activating the corresponding receptors by binding thereto. As a result of such receptor activation, various diseases or pathogenic changes can be induced and/or accompanied. For example, it is possible that activation of receptors modulates the production of messenger molecules. However, the autoantibodies may also damage receptors of cells which are responsible for essentially normal blood flow in limbs. In contrast to the effect of agonistic autoantibodies, the function of attacking and damaging the corresponding target by chronic stressing - usually observed with antibodies - is the predominant one in non-agonistic autoantibodies. However, this does not exclude activation or deactivation of the receptors.

In a preferred embodiment of the invention the peptide additionally comprises amino groups, amides, acetyl groups, biotin groups, markers, spacers and/or linkers.

By virtue of such structures, the peptide can be used with advantage in various fields of diagnosis and therapy of autoimmune diseases of the optic nerve involving loss of retinal ganglion cells. Various ways of modifying peptides for various applications are well-known to those skilled in the art. If, for example, the peptides are envisaged to be administered as a drug, the structure of the peptides has to be changed in an specific fashion, as is well-known to those skilled in the art. However, the peptides may also be bound to the supporting material of an affinity column in order to be used in the purification of body fluids, especially blood; binding of the peptides to a matrix requires specific structural modifications of the peptides according to the invention, and such modifications are also well-known to those skilled in the art or can be determined using routine tests.

The amino acids of the peptides may represent gene encoded peptides with similar properties as well as retro-inverso peptides with D-amino acids (small letters) with similar properties, available by biochemical or chemical peptide synthesis.

As is well-known to those skilled in the art, some amino acids have analogous physicochemical properties so that these amino acids advantageously can be replaced by each other. For example, these include the group of nonpolar (hydrophobic) amino acids (a) glycine, alanine, valine, leucine and/or isoleucine; or the hydroxy amino acids (b) serine, threonine and/or tyrosine, the amides of amino dicarboxylic acids (c) asparagine and glutamine, the amino dicarboxylic acids (d) aspartic acid and glutamic acid; the basic amino acids (e) lysine, arginine and/or ornithine as well as the group of aromatic amino acids (f) phenylalanine, tyrosine and/or tryptophan. Another aspect is the replacement of amino acids by structural similar amino acids. For example this is the case in the group with a β-functional group (g) cysteine, methionine, serine, α- aminobutyric acid and selenocysteine as well as the turn-inducing group (h) praline, 1-amino-2-carboxy cyclohexane, pipecolic acid and an ortho-aminobenzoic acid. Amino acids within one and the same group (a-h) can be replaced with one another. In all cases, peptide sequences will have a sufficient homology to be an analogous to an amino acid sequence of the peptides of the invention. Furthermore, the amino acids can be replaced by modified amino acids or specific enantiomers. Further modifications are possible in accordance with the teaching of WO 99/62933 or WO 02/38592. Examples for such modified sequences are SVQSFAN and ANDSCSE. In both examples several amino acids of the peptides of the invention were replaced by other amino acids with similar physio-chemical or structural properties. Another example is the sequence SLN-Abu-YAN-GKK. Additional to the examples before, here for X9 is coupled the group GKK. It is obviously known to a person skilled in the art that it is possible to provide functionally analogous means for the peptides and nudeic acids of the invention. In the meaning of the invention, "functionally analogous means" are means which are functionally equivalent or equivalent to the peptides and nudeic acids of the invention. In order to check whether a sequence is functionally equivalent (= equivalent) to the peptides or nudeic acids of the invention, one must check whether the functionally analogous/equivalent sequence of the peptide or of the nudeic acid produces essentially the same function in essentially the same way with essentially the same outcome. That is, the outcome produced with the peptides or nudeic acids of the invention may also be produced with the functionally analogous/equivalent means. A person skilled in the art can therefore easily and quickly verify whether a functionally analogous/equivalent sequence provided by them is covered by the invention. The functionally analogous sequence is covered by the invention if it may be used for solving the problem of the invention and if the solving of the problem of the invention can be carried out in essentially the same way as is claimed by the invention.

It should be understood that various alternatives to the embodiments of the invention described herein may be employed in practicing the invention. It is intended that the claims of the invention define the scope of the invention and that the method and apparatus within the scope of these claims and their equivalents be covered thereby.

Those skilled in the art will therefore understand all those molecules as equivalent which he may apply without this having an effect on the solution of the problem of the invention. They know that the claimed sequences must not be modified by changes, substitutions or deletions in a way that they cannot be used for solving the problem of the invention.

In another preferred embodiment the peptide comprises a linker and/or a spacer selected from the group comprising α-aminocarboxylic acids as well as homo- and heterooligomers thereof, α,ω-aminocarboxylic acids and branched homo- or heterooligomers thereof, other aliphatic and/or aromatic amino acids, as well as linear and branched homo- or heterooligomers (peptides); amino-oligoalkoxyalkylamines; maleinimidocarboxylic acid derivatives; oligomers of alkylamines; 4-alkylphenyl derivatives; 4-oligoalkoxyphenyl or 4-oligoalkoxyphenoxy derivatives; 4-oligoalkylmercaptophenyl or 4-oligoalkyl-mercaptophenoxy derivatives; 4-oligoalkylaminophenyl or 4-oligoalkylaminophenoxy derivatives; (oligoalkylbenzyl)phenyl or 4-(oligoalkylbenzyl)phenoxy derivatives, as well as 4-(oligoalkoxybenzyl)phenyl or 4-(oligoalkoxybenzyl)phenoxy derivatives; trityl derivatives; benzyloxyaryl or benzyloxyalkyl derivatives; xanthen-3-yloxyalkyl derivatives; (4-alkylphenyl)- or ω-(4-alkylphenoxy)alkanoic acid derivatives; oligoalkylphenoxyalkyl or oligoalkoxyphenoxyalkyl derivatives; carbamate derivatives; amines; trialkylsilyl or dialkylalkoxysilyl derivatives; aliphatic or aromatic mono- or oligomercapto derivatives; alkyl or aryl derivatives and/or combinations thereof; other possible structures have been described in EP 1214 350. The following sequence is an example: GLN-Cys(Maleinimidopropionic acid)-TYAN. In this sequence a peptide of the invention several amino acids were replaced by other with similar properties as described above. Additionally, the SH group of the cysteine reacts with the double bound of the maleinimide group to form a covalent bound to a linker molecule. This linkage is could couple to another molecule or a solid phase.

Also claimed according to the invention are solid phases for affinity chromatography or solid phase extraction constisting of organic, inorganic, synthetic polymers or of mixed polymers, preferably crosslinked agarose, cellulose, silica gel, polyamide and polyvinyl alcohols, which are optionally chemically activated, with peptides according to the invention immobilized on the surface of the solid phase.

In the solid phase according to the invention, the peptides are bound to the solid support phase preferably covalently or by adsorption. In another preferred embodiment of the solid phases according to the invention, the peptides are distanced from the support surface by linkers/spacers.

The invention relates to a method for the adsorption of immunoglobulins to a solid phase, wherein the peptides according to the invention are bound to a solid phase usual in affinity chromatography or to mobile solid phases, and the immunoglobulin-containing samples to be adsorbed are contacted, in particular, with the solid phases according to the invention.

Preferably, the method for the adsorption of immunoglobulins is performed on a solid phase, wherein the peptides according to the invention are bound to a solid phase usual in affinity chromatography or to mobile solid phases, and the immunoglobulin-containing samples to be adsorbed are anticoagulanttreated human blood plasma or human whole blood and are contacted with the solid phases according to the invention.

The method according to the invention is suitable, in particular, for the adsorption of immunoglobulins from immunoglobulin-containing samples which contain auto-antibodies related to autoimmune dises, especially rheumatoid diseases, multiple sclerosis, myasthenia gravis and other auto-antibody-mediated diseases.

The method according to the invention is advantageously performed with a device according to the invention for removing immunoglobulins from immunoglobulin-containing samples on solid phases, wherein the device contains a solid phase according to the invention, and means for the entry of immunoglobulin-containing samples are provided.

The invention also relates to the use of the peptides according to the invention, the solid phases according to the invention and the devices according to the invention for removing immunoglobulins from immunoglobulin-containing samples.

According to another particularly preferred embodiment of the invention, the peptide is selected from the group comprising:
a) a peptide essentially containing the amino acid sequence according to claim 1, preferred AINCYAN and/or ANETCCD; or
b) a peptide according to amino acid sequence a) which is modified by branch or extension with the same or another peptide according to amino acid sequence a), b) or c) to form a homooligomeric or heterooligomeric peptide. The coupling could result by a direct bond to amino acids of the sequences or by an indirect bond to amino acids of the sequences using a linker or spacer.

Examples of sequences are as follows:

Ser - Leu - Asn - Cys - Tyr - Ala - Asn
Ala - Asn - Glu- Thr - Cys - Ser - Glu

In this example several amino acids of peptides of the invention were replaced by amino acids with similar properties. Two peptides were coupled together via a disulfide bridge to form a heterodimer. Such heterodimer is able to represent two different areas of one epitope. Due to the combination of both peptides an increasing of the activity is possible without using other sequences than these of the peptides of the invention.

Another example is following structure:

Ala - Asn - Glu - Thr - Cys - Lys - Cys - Asp
S - CH2 - CH2

Here two parts of one peptide of the invention were linked together by an inserted lysine. Additionally, both cysteines were brigded via a thioethane spacer. With such construct the stabilisation of a specific structure is possible.

Preferred sequences according to the claims are as follows:
1) Ala-Ile-Asn-Cys-Tyr-Ala-Asn
   original sequence 1 of claim 15a
2) Ala-Asn-Glu-Thr-Cys-Cys-Asp
   original sequence 2 of claim 15a
3) D-Asn-D-Ala-D-Tyr-D-Cys-D-Asn-D-Ile-Gly
   retro-inverso peptide according to original sequence 1 of claim 1
4) D-Asp-D-Cys-D-Cys-D-Thr-D-Glu-D-Asn-D-Ala
   retro-inverso original sequence 2 of claim 1
5) 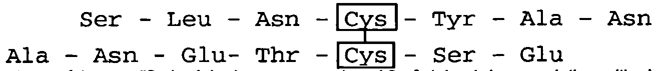
Ser - Leu - Asn - Cys - Tyr - Ala - Asn
Ala - Asn - Glu- Thr - Cys - Ser - Glu
[?]
 dimer of the modified original sequences 1 and 2 of claim 1, in association with claims 3b (and 3d)
6) Gly - Leu - Asn - Cys(Maleinimidopropionic acid)-Tyr-Ala-Asn with linker coupled to Cys modified original sequence 1 of claim 1, in association with claim 2
7) Ser-Leu-Asn-Abu-Tyr-Ala-Asn-Gly-Lys-Lys
   with C-terminal coupled linker and otherwise modified original sequence 1 of claim 1, in association with claim 2
8) Cys - Ala - Asn - Glu - Thr - Cys - Ala - Glu by cyclization and otherwise modified original sequence 2 of claim 1, in association with claim 11
9) Ser - Leu - Asn - Cys - Trp - Asn - Gln by head-to-tail cyclization and otherwise modified original sequence 1 of claim 1, in association with claim 11
10) 
Ala - Asn - Glu - Thr - Cys - Lys(Cys-Asp)
S - CH2 - CH2
 by insertion and cyclisation via spacer modified original sequence original sequence 2 of claim 1, in association with claims 2, 3c and 3d

In a distinctive embodiment of the invention the peptide which has sufficient homology to be functionally analogous to one of the following amino acids of claim 1, preferred GLQSWGQ, SVEATSE, VADMSVD, AQNTCCN, GNQSSSQ, SEEYAHE, more preferred AINCYAN and/or ANETCCD has at least 90% homology thereto. In another preferred embodiment said amino acid sequences have at least 90% homology to one of the following amino acid sequences of claim 1, preferred GLQSWGQ, SVEATSE, VADMSVD, AQNTCCN, GNQSSSQ, SEEYAHE, more preferred AINCYAN and/or ANETCCD.

In an especially preferred embodiment of the invention the peptide essentially consists of the amino acid sequence of claim 1, preferred GLQSWGQ, SVEATSE, VADMSVD, AQNTCCN, GNQSSSQ, SEEYAHE, more preferred AINCYAN and/or ANETCCD. More specifically, the peptide consists of modifications of said sequences, which are obtained according to WO 99/62933 and WO 02/38592, and it goes without saying that these peptides bind autoantibodies in the meaning of the invention.

In another preferred embodiment of the invention the peptide is used or employed as a therapeutic active substance. In the meaning of the invention, use as a therapeutic active substance means use of the peptide in the entire field of medicine.

In another particularly preferred embodiment of the invention the peptide is bound by specific antibodies of patients with glaucoma, especially by autoantibodies. At a defined quantity ratio of peptide and auto-antibody, which is well-known to those skilled in the art, autoantibody-peptide complexes will form which, for example, undergo precipitation or exhibit specific reaction behavior in a way so as to allow elimination or reduction of the autoantibodies.

In another preferred embodiment of the invention the peptide, in particular, is immobilized. In the meaning of the invention, immobilization is understood to involve various methods and techniques to fix the peptides on specific carriers, e.g. according to WO 99/56126 or WO 02/26292. For example, immobilization can serve to stabilize the peptides so that their activity would not be reduced or adversely modified by biological, chemical or physical exposure, especially during storage or in single-batch use. Immobilization of the peptides allows repeated use under technical or clinical routine conditions; furthermore, a sample - preferably blood components - can be reacted with at least one of the peptides according to the invention in a continuous fashion. In particular, this can be achieved by means of various immobilization techniques, with binding of the peptides to other peptides or molecules or to a carrier proceeding in such a way that the three-dimensional structure - particularly in the active center mediating the interaction with the autoantibodies - of the corresponding molecules, especially of said peptides, would not be changed. Advantageously, there is no loss in specificity to the autoantibodies of the patient as a result of such immobilization. In the meaning of the invention, three basic methods can be used for immobilization:
(i) Crosslinking: in crosslinking, the peptides are fixed to one another without adversely affecting their activity. Advantageously, they are no longer soluble as a result of such crosslinking.
(ii) Binding to a carrier: binding to a carrier proceeds via adsorption, ionic binding or covalent binding, for example. Such binding may also take place inside microbial cells or liposomes or other membranous, dosed or open structures. Advantageously, the peptides are not adversely affected by such fixing. For example, multiple or continuous use of carrier-bound peptides is possible with advantage in clinics in diagnosis or therapy.
(iii) Inclusion: inclusion in the meaning of the invention especially is inclusion in a semipermeable membrane in the form of gels, fibrils or fibers. Advantageously, encapsulated peptides are separated from the surrounding sample solution by a semipermeable membrane in such a way that interaction with the autoantibodies or fragments thereof still is possible.

Various methods are available for immobilization, such as adsorption on an inert or electrically charged inorganic or organic carrier. For example, such carriers can be porous gels, aluminum oxide, bentonite, agarose, starch, nylon or polyacrylamide. Immobilization proceeds via physical binding forces, frequently involving hydrophobic interactions and ionic binding. Advantageously, such methods are easy to handle, having little influence on the conformation of the peptides. Advantageously, binding can be improved as a result of electrostatic binding forces between the charged groups of the peptides and the carrier, e.g. by using ion exchangers such as Sephadex.

Another method is covalent binding to carrier materials. In addition, the carriers may have reactive groups forming homopolar bonds with amino acid side chains. Suitable functional side chain groups in peptides are carboxy, hydroxy and hydrosulfide groups and especially the terminal amino groups of lysines. Aromatic groups offer the possibility of diazo coupling. The surface of microscopic porous glass particles can be activated by treatment with silanes and subsequently coated with peptides. For example, hydroxy groups of natural polymers can be activated with bromocyanogen and subsequently coupled with peptides. Advantageously, a large number of peptides can undergo direct covalent binding with polyacrylamide resins. Inclusion in three-dimensional networks involves inclusion of the peptides in ionotropic gels or other structures well-known to those skilled in the art. More specifically, the pores of the matrix are such in nature that the peptides are retained, allowing interaction with the target molecules. In crosslinking, the peptides are converted into polymer aggregates by crosslinking with bifunctional agents. Such structures are gelatinous, easily deformable and, in particular, suitable for use in various reactors. By adding other inactive components such as gelatin in crosslinking, advantageous improvement of mechanical and binding properties is possible. In microencapsulation, the reaction volume of the peptides is restricted by means of membranes. For example, microencapsulation can be carried out in the form of an interfacial polymerization. Owing to the immobilization during microencapsulation, the peptides are made insoluble and thus reusable. In the meaning of the invention, immobilized peptides are all those peptides being in a condition that allows reuse thereof. Restricting the mobility and solubility of the peptides by chemical, biological or physical means advantageously results in lower process cost, particularly when eliminating autoantibodies from blood components.

In another preferred embodiment of the invention the peptide is bound to a solid phase. Binding the peptide to the solid phase may proceed via a spacer. All those chemical compounds can be used as spacers which have the structural and functional preconditions suitable for the function of a spacer, provided they do not modify the binding behavior in such a way that binding of the autoantibody with the peptide would be adversely affected.

The invention also relates to recognition molecules directed against the agonistic autoantibodies which interact with the peptides of the invention. These agonistic autoantibodies are directed against the beta-2 receptor. The interaction or reaction or binding of the autoantibody is a specific antigen-antibody-interaction. In a preferred embodiement the recognition molecules are antibodies, antisense constructs and/or chelating agents and/or aptamers or Spiegelmers. The recognition molecules according to the invention can be antibodies directed against the autoantibodies inducing e.g. diseases of the optic nerve, especially glaucoma; it is also possible that the recognition molecule is a peptidomimetic. To understand what a Spiegelmer is, it is important to first explain the concept of aptamers. An aptamer is a nucleic acid structure that can bind to a target molecule conceptually similar to an antibody that recognizes an antigen (Ellington & Szostak, 1990). Based on the SELEX (Systematic Evolution of Ligands by EXponential enrichment) process (Tuerk & Gold, 1990), aptamers can be identified from huge combinatorial nucleic acids libraries of up to > 10¹⁵ different sequences. The sequences in the library are comprised of a central randomized region flanked by fixed sequences that permit amplification by polymerase chain reactions (PCR). The library size depends on the length of the random portion of the molecule, creating a diverse universe of molecules ready for screening. The aptamers capable of binding to a target are then selected by incubating the libraries with the immobilized target, washing extensively, then eluting the bound aptamer. After amplification by PCR, these binding molecules are re-selected, eluted and amplified. By repeating this procedure multiple times, and eluting bound aptamers under progressively more stringent conditions, the molecules with greatest affinity and specificity are selected.

Aptamers have binding characteristics similar to peptides or antibodies, with affinities in the low nanomolar to the picomolar range. However, there are several drawbacks to aptamers as useful therapeutic products. As relatively small molecules, aptamers demonstrate circulating half-lives in vivo in the order of minutes. This situation can be addressed by attaching large inert molecules to aptamers (e.g. polyethylene glycol) to reduce their elimination via the kidney and increase their presence in the circulation. Finally, aptamers, as natural nucleic acid polymers, are prone to rapid degradation by nucleases that are present in all tissues in the body. To overcome this latter problem, methods of creating nuclease-resistant molecules that retained the binding capabilities of aptamers needed to be created. The answer to this latter problem was solved by the discovery and development of Spiegelmers (Klussmann et al., 1996; Vater & Klussmann, 2003). These molecules, which are biostable aptamers, have all of the diversity characteristics of aptamers but possess a structure that prevents enzymatic degradation. While aptamers are created from the natural D-nucleotides, which are recognized by the nucleic acid degrading enzymes, a molecule synthesized as the mirror image L-oligonucleotide will not be degraded by any nuclease since there are no such enzymes in the body capable of interacting with these unnatural molecules. As such, the amplification methods used to create large aptamer libraries cannot be employed to synthesize the L-nucleic acid-containing molecules. However, in the state of the art there are known methods used to form high affinity L-oligonucleotide aptamers that possess outstanding binding affinities and functionality. These molecules are termed "Spiegelmer" from the German "Spiegel" or mirror. Based on the simple concept that if an aptamer binds its natural target, the mirror image of the aptamer will identically bind the mirror image of the natural target. If the process of aptamer selection is carried out against the mirror image target, an aptamer against this unnatural mirror image will be obtained. The corresponding mirror image nudeic acid (L-oligonucleotide) of this aptamer, or the Spiegelmer, should-and will - now bind to the natural target ligand with similar binding characteristics as the aptamer itself. More important, this Spiegelmer is now resistant to nuclease degradation. Spiegelmers possess the high affinity binding characteristics of the best aptamers and antibodies in the low nanomolar and picomolar range, while defying enzymatic degradation that severely limits the utility of aptamers. Data indicate that Spiegelmers are stable in human plasma for over 60 hours at 37°C, while non-modified RNA aptamers are degraded in seconds under the same conditions. Results in animals indicate that a similar stability can be expected in vivo as well. Spiegelmers should not be confused with antisense RNAs in that they do not directly interfere with protein synthesis of their target molecules. They are designed to bind specifically to extracellular molecules, either a receptor or its ligand, similar to the behavior of a monoclonal antibody, aptamer or peptide. However, recent findings demonstrate that Spiegelmers are capable of entering cells and interfering with intracellular processes as well. Finally, Spiegelmers appear to be non-immunogenic, even under the most inductive conditions for antibody formation in rabbits. This situation is of critical importance if Spiegelmer therapy is to be used repeatedly to treat chronic diseases. It is therefore preferred in the context of the present invention, to detect substances which bind or react or interact with the agonistic autoantibodies against the beta-2 receptor. These Spiegelmers can be used as peptidomimetic. It is also preferred to detect substances which block Spiegelmers that block the antigen binding site of the agonistic autoantibodies.

Also claimed by the invention are RNAi-pharmaceuticals for blocking the plasma cells which produce the agonistic autoantibodies directed against the beta-2 receptor.

The invention also relates to a pharmaceutical composition comprising the inventive nucleic acid molecules, vectors, host cells, peptides and/or recognition molecules, optionally together with a pharmaceutically tolerable carrier. In particular, the pharmaceutical composition can be used as a drug. To this end, it is possible, for example, to modify the peptides by means of cyclization or other procedures well-known to those skilled in the art such that destruction thereof by endogenous peptide-degrading structures, e.g. serum proteases, is prevented. By using the peptides or recognition molecules according to the invention, *in vivo* or *ex vivo* neutralization of autoantibodies is possible. In *in vivo* neutralization, the drugs are administered directly to the patient; in *ex vivo* neutralization, the blood is conducted out of the body e.g. via a loop, e.g. in the form of a tube circulation, subsequently contacted with the drug and, following neutralization of the autoantibodies, resupplied into the organism, i.e., the patient Regarded as drugs in the meaning of the invention are compositions for therapeutic and prophylactic purposes, as well as pharmaceutical compositions usable as diagnostic agents.

According to the invention, drugs or pharmaceutical compositions - used in a synonymous fashion herein - are substances and formulations of substances intended to cure, alleviate or avoid diseases, illness, physical defects or pathological affection by application on or in the human body. According to the invention, medical adjuvants are substances used as active ingredients in the production of drugs. Pharmaceutical-technical adjuvants serve to suitably formulate the drug or pharmaceutical composition and, if required during the production process only, can even be removed thereafter, or they can be part of the pharmaceutical composition as pharmaceutical tolerable carriers. Examples of pharmaceutically tolerable carriers will be given below. Drug formulation or formulation of the pharmaceutical composition is optionally effected in combination with a pharmaceutically tolerable carrier and/or diluent. Examples of suitable pharmaceutically tolerable carriers are well-known to those skilled in the art and comprise e.g. phosphate-buffered saline, water, emulsions such as oil/water emulsions, various types of detergents, sterile solutions, and so forth. Drugs or pharmaceutical compositions comprising such carriers can be formulated by means of well-known conventional methods. These drugs or pharmaceutical compositions can be administered to an individual at a suitable dose, e.g. in a range of from 0,01 µg to 10 g of peptides per day and patient Doses of from 1 µg to 1 g are preferred. Preferred is administration of doses as small in number and as low as possible, preferably a single dose. Administration can be effected on various routes, e.g. intravenous, intraperitoneal, intrarectal, intragastrointestinal, intranodal, intramuscular, local, but also subcutaneous, intradermal or on the skin or via mucosa. Administration of nudeic acids encoding the peptide according to the invention can also be effected in the form of a gene therapy, e.g. via viral vectors. The kind of dosage and route of administration can be determined by the attending physician according to clinical factors. As is familiar to those skilled in the art, the kind of dosage will depend on various factors such as size, body surface, age, sex, or general health condition of the patient, but also on the particular agent being administered, the time period and type of administration, and on other medications possibly administered in parallel. Those skilled in the art will also be familiar with the fact that the concentration of autoantibodies can be diagnosed first, using the peptides according to the invention, in order to determine the required concentration of drug.

More specifically, the pharmaceutical compositions or drugs comprise a pharmacological substance which includes one or more peptides or recognition molecules of the invention, or/and nucleic acid molecules encoding same, in a suitable solution or administration form. Administration thereof can be effected either alone or together with appropriate adjuvants described in connection with drugs or pharmaceutical compositions, or in combination with one or more adjuvants, e.g. QS-21, GPI-0100 or other saponines, water-oil emulsions such as Montanide adjuvants, polylysine, polyarginine compounds, DNA compounds such as CpG, Detox, bacterial vaccines such as typhoid vaccines or BCG vaccines, salts such as calcium phosphates, and/or other suitable material enhancing the effect, preferably immunostimulatory molecules such as interleukins, e.g. IL-2, IL-12, IL-4 and/or growth factors such as GM-CSF. They are mixed with the peptides or recognition molecules of the invention according to well-known methods and administered in suitable formulations and dosages. Formulations, dosages and suitable components are well-known to those skilled in the art.

Obviously, the pharmaceutical composition or drug can also be a combination of two or more of the inventive pharmaceutical compositions or drugs, as well as a combination with other drugs, such as antibody therapies, chemotherapies or radiotherapies, suitably administered or applied at the same time or separately in time. The production of the drugs or pharmaceutical compositions proceeds according to per se known methods.

The invention also relates to a kit comprising the nudeic acid molecule, the vector, the host cell, the peptide and/or the recognition molecule, optionally together with instructions or information as to the pharmaceutical provision or the procedure of therapeutical treatment. For example, the information can be an instruction leaflet or other medium providing the user with information in which therapeutical procedure the above-mentioned substances should be used. In particular, the instruction leaflet includes detailed and/or important information about the therapeutical treatment. Obviously, the information need not necessarily be in the form of an instruction leaflet, and the information may also be imparted via the Internet.

The invention also relates to an apparatus for chromatography, which includes the peptides according to the invention. In a preferred embodiment the peptides are bound to a solid phase or an apparatus which catches from blood or plasma extracellular loop 2 peptides according to the human ß2 receptor or antibody binding peptido mimetica fixed to nanoparticles, magnetic or paramagnetic particles or micro-spheres of cellulosis and other polymeric matrix molecules.

In another preferred embodiment the peptides are bound to a solid phase within the chromatography system.

In particular, the apparatus according to the invention (apparatus which features the peptides according to the invention) can be used to eliminate the autoantibodies from fluids of a patient or to neutralize the autoantibodies. This method is known to those skilled in the art under the term of immunoadsorption and/or plasma apheresis therapy. With the aid of immunoadsorption, immunoglobulins are removed from the blood or serum of a glaucoma patient. Advantageously, this immunoadsorption treatment can be conducted in a stationary and ambulant fashion. It can be envisaged that the apparatus, particularly the so-called adsorber, is part of an extracorporeal blood circulation. To this end, blood is taken continuously or discontinuously from a patient's major vessel, particularly from an arm vein, and separated into single components, such as cellular and humoral components, using filtration or centrifugation. In particular, one essential blood component obtained in this fashion is blood plasma. Advantageously, the blood plasma can be passed through the apparatus of the invention and, following adsorption of the autoantibodies, returned into the patient, particularly through another vein of arms or legs, together with previously separated blood components, especially cellular components. It can also be envisaged that the peptides are immobilized on a Sepharose matrix. The matrix can be placed in a container having a volume of e.g. 10 to 400 ml. Thereafter, the blood plasma of the patient can be passed over the matrix where the autoantibodies will be bound, thus allowing elimination thereof from the blood plasma. Those skilled in the art will be familiar with various ways of providing such solid phase-fixed peptides, e.g. in the form of (i) regeneratable adsorption columns, (ii) double columns and (iii) disposable columns. The diverse wash and elution solutions, permitting high efficiency of treatment, can easily be determined by a person skilled in the art by using routine tests. By providing the teaching according to the invention, particularly the peptides of the invention, various ways of employing the peptides *in vivo, ex vivo* and *in vitro* in prophylaxis, diagnosis, therapy and aftercare of glaucoma-induced, autoantibody-mediated diseases are disclosed to a person skilled in the art.

The invention also relates to a method for the treatment of glaucoma by binding and/or removing autoantibodies by means of the peptides of the invention bound to a solid phase.

In a preferred embodiment of the invention the autoantibodies are directed against the human beta-2-adrenergic receptor.

The invention also relates to the use of the nudeic acid molecules of the invention, the host cells of the invention, the vector of the invention, the peptides of the invention, the recognition molecules of the invention, the pharmaceutical composition of the invention, the kit of the invention and the apparatus of the invention in the prophylaxis, diagnosis, therapy, follow-up and/or aftercare of glaucoma.

The invention also relates to the use of the nudeic acid molecules of the invention, the host cells of the invention, the vector of the invention, the peptides of the invention, the recognition molecules of the invention, the pharmaceutical composition of the invention, the kit of the invention and the apparatus of the invention in the production of a drug for the treatment of glaucoma.

In a preferred embodiment of the invention the glaucoma is a primary open angle glaucoma (POAG), a normal tension glaucoma (NTG), an acute angle-closure glaucoma, a primary congenital glaucoma and/or a secondary glaucoma.

According to the invention, the most common type, primary open angle glaucoma (POAG), frequently has no symptoms and has been nicknamed "the sneak thief of sight". One factor may be a relative obstruction on the outflow of aqueous humour from the eye. Aqueous humour is produced by the ciliary body of the eye, and then flows through the pupil and into the anterior chamber. The trabecular meshwork then drains the humour to Schlemm's canal, and ultimately to the venous system. All eyes have some intraocular pressure, which is caused by some resistance to the flow of aqueous through the trabeculum and Schlemm's canal. If the intraocular pressure (IOP) is too high, (>21.5 mm Hg), the pressure exerted on the walls of the eye results in compression of the ocular structures. However, other factors such as disturbances of blood flow in the optic nerve head may interact with IOP to affect the optic nerve. In one third of cases of POAG there is statistically normal IOP. This is called normal tension glaucoma (NTG). Because optic nerve examination and perimetry testing are not always done in addition to IOP measurement in those at risk, NTG is underdiagnosed and the condition presents late.

According to the invention, another type, acute angle-closure glaucoma, is characterized by an acute rise in the intraocular pressure. This occurs in susceptible eyes when the pupil dilates and blocks the flow of fluid through it, leading to the peripheral iris blocking the trabecular meshwork. Acute angle-closure glaucoma can cause pain and reduced visual acuity (blurred vision), and may lead to irreversible visual loss within a short time. This is an ocular emergency requiring immediate treatment Many people with glaucoma experience halos around bright lights as well as the loss of sight characterized by the disease.

In the meaning of the invention, primary congenital glaucoma or buphthalmos is a rare genetic disease affecting infants. Newborns present with enlarged globes and clouded corneas. It is thought that reduced trabecular permeability is the cause of increased intraocular pressure. Surgery is the treatment.

In the meaning of the invention, secondary glaucoma occurs as a complication of various medical conditions such as eye surgery, advanced cataracts, eye injuries, some eye tumors, uveitis, diabetes or use of corticosteroid drugs.

The invention also relates to the use of the inventive peptides and nudeic acids or host cells, the pharmaceutical composition of the invention, the kit of the invention and/or the apparatus of the invention in drug screening. For example, drug screening may comprise the identification of substances, particularly peptides, proteins, carbohydrates and/or lipids, which interact with beta-2-adrenergic-receptor-autoantibodies.

Interaction, for example, can be binding to said peptides, but also activation or inhibition of or by peptides. Accordingly, a drug can be e.g. a structure binding to the autoantibodies or - in another embodiment of the invention - to the peptide structures in the body of a patient, and consequently to the loops, thus competing with the autoantibodies for a binding site. By virtue of the disclosure of the teaching according to the invention, especially the disclosure with respect to the connection of disease and binding site of the autoantibodies, a person skilled in the art will be capable of screening various drugs. Drug screening based on disclosed targets is part of the general knowledge of a person skilled in the art and is effected using routine tests; reference is made to the corresponding standard textbooks of molecular biology and pharmacology.

The invention also relates to a method for the treatment of an autoimmune disease by binding and/or removal of autoantibodies by means of the inventive peptides bound to a solid phase. The autoantibodies are bound, complexed and/or neutralized on the solid phase by means of the peptides bound to the solid phase.

In a preferred embodiment of the invention, autoantibodies directed against the beta-2-adrenergic receptor are bound, complexed and/or neutralized by means of the above-mentioned inventive materials and products, apparatus, particularly the chromatography apparatus, and peptides. For example, the peptides can be used in the detection of autoantibodies in serums of patients, using an ELISA or other immunological detection methods well-known to those skilled in the art. For detection, it can be advantageous, for example, when the autoantibodies are bound by biotinylated or otherwise coupled peptides and separated by streptavidin-coupled supports such as magnetic particles or plates. Such a method has been described in DE 102 56 897.9. More specifically, the separated autoantibodies are detected using igG subtype-specific labelled antibodies. In the event of a primary open angle glaucoma, the antibodies are detected using particularly IgG₃ subtype-specific labelled antibodies.

The invention relates also to the use of a peptide according to claim 1 in the manufacture of a column coupled to said ligand for the treatment of a patient suffering from glaucoma, said treatment comprising passing plasma of the patient, over the column under conditions which effect the binding of said specific ligand to immunoglobulin in the patient's plasma, thereby removing a significant portion of the immunoglobulin from the patient's plasma, and reinfusing the plasma to the patient.

Additionally, the invention relates also to the treatment of a patient suffering from glaucoma, said treatment comprising the steps of:
(a) providing a column coupled to a peptide according to claim 1,
(b) passing plasma of the patient over the column under conditions which effect the binding of said peptide to immunoglobulin in the patient's plasma, thereby removing a significant portion of the immunoglobulin from the patient's plasma, and
(c) reinfusing said plasma to the patient.

In another preferred embodiment of the treatment of a patient, the peptide of claim 1 recognizes antibodies directed against tissue of the optic nerve. Preferably, the peptide is an antigen-mimicking molecule.

Preferably, the peptide is a synthesized peptide mimicking a sequence of a receptor structure.
In another preferred embodiment of the treatment of a patient, the receptor is the beta-2-adrenergic receptor.

Preferably, the autoantibodies are directed against a molecule selected from the group consisting of beta-2-adrenergic receptors.

In another preferred embodiment of the treatment of a patient, the invention is characterized by a parallel or subsequent combination with β-blockers or intravenous immunoglobulin.

The teachings of the present invention are characterised by the following features:
- departure from the beaten track
- a new perception of the problem
- satisfaction of a long-felt need or want
- hitherto all efforts of experts were in vain
- the simplicity of the solution, which proves inventive action, especially since it replaces a more complex doctrine
- the development of scientific technology followed another direction
- the achievement forwards the development
- misconceptions among experts about the solution of the according problem (prejudice)
- technical progress, such as: improvement, increased performance, price-reduction, saving of time, material, work steps, costs or resources that are difficult to obtain, improved reliability, remedy of defects, improved quality, no maintenance, increased efficiency, better yield, augmentation of technical possibilities, provision of another product, opening of a second way, opening of a new field, first solution for a task, spare product, alternatives, possibility of rationalisation, automation or miniaturisation or enrichment of the pharmaceutical fund
- special choice; since a certain possibility, the result of which was unforeseeable, was chosen among a great number of possibilities, it is a patentable lucky choice
- error in citations
- young field of technology
- combined invention; a combination of a number of known elements, with a surprising effect
- licensing
- praise of experts and
- commercial success

Said advantages are shown especially in the preferential embodiments of the invention.

Without intending to be limiting, the invention will be explained in more detail with reference to the examples.

### Examples

### Methods for identifying agonistic antibodies against the ß2 adrenergic receptor

*Cell culture.* Isolation and cultivation of neonatal heart cells were performed as described previously (Wallukat, G., and Wollenberger, A. 1987. Effects of the serum gammaglobulin fraction of patients with allergic asthma and dilated cardiomyopathy on chronotropic β-adrenoceptor function in cultured rat heart myocytes. Biomed. Biochim. Acta. 78:634-639)

Briefly, single cells were dissociated from the minced ventricles of Wistar rats (1-2 days old) with a 0.25% solution of crude trypsin and were cultured as monolayers with a density of 800 cells/mm2 in Halle SM 20-I medium equilibrated with humidified air. The medium contained 10% heat-inactivated FCS and 2 µmol/1 fluorodeoxyuridine (Serva, Heidelberg, Germany) the latter to prevent proliferation of nonmuscle cells.

On the third or fourth days, the cells were incubated for 2 h in 2 ml fresh serum-containing medium. Seven to 10 selected cells or synchronously contracting cell clusters per flask were counted for 15 s. This procedure was repeated twice in different cultures to yield results representing a total of up to 30 cells for each sample.

Immunoglobulin fractions, agonist and antagonist drugs, peptides, etc. were added singly or cumulatively as indicated. The basal contraction rate of the spontaneously beating cardiomyocytes was 162 ± 4 min.

*Preparation of the immunoglobulin fraction.* The immunoglobulin fraction was isolated from 1- to 2-ml serum samples by ammonium sulfate precipitation at a saturation of 40%. The precipitates were washed and dissolved in dialysis buffer (154 mmol/l NaCl, 10 mmol/l sodium phosphate; pH 7.2). The procedure of precipitating, washing, and dissolving was repeated twice.

Finally, the immunoglobulins were taken up in 1 ml PBS (pH 7.2) and dialyzed at 4°C for 30 hours against 1 l of dialysis buffer. The buffer was changed five times during dialysis. For detection of autoantibodies, the immunoglobulin fractions were added at a dilution of 1:20 or 1:40 to the flasks.

After the dialysis, the IgG fractions feature a concentration of 10-15 mglml, a maximum of 10 µg/ml of which are specific receptor antibodies. 50 µl of this IgG solution are pre-incubated with the peptides with the following concentration of peptides: 5 µl of a buffered solution of 100 µg/ml per 50 µl IgG solution. This mixture is pre-incubated at room temperature for 30 minutes and subsequently filled into the cell culture vials, after the former nutrient solution has been removed by pipet. The amount of the medium is 2 ml. The final concentration therefore is of 0.5-0.75 mg/ml for the IgG and 0.5 µg for the peptides. If the molecular weight of the peptide (10 mer) is set with 1 KD and that of the IgG is set with 150 KD, there is an excess of agonistic auto-antibodies.

For the neutralization experiments, synthetic peptides corresponding to the sequence of the three extracellular loops of the human ß2 adrenegic receptor was added in excess (0.5 µg in 50 µl) to 50 µl of the immunoglobulin fraction. The mixtures were shaken and incubated at room temperature for 1 h. The 100-µl samples were then added to neonatal rat heart muscle cells cultured in 2 ml medium to a final dilution of 1:40. The beating rate was counted for 15 s, 60 min after the addition of the peptide/immunoglobulin mixture.

For identifying the autoantibody neutralizing epitopes synthetic overlapping peptides corresponding to the sequence of the second extracellular loop of the human ß2 adrenoceptor was added to the immunoglobulins. After 1 h incubation at room temperature the samples were used as described above.

*Patients.* 48 patients with glaucoma (primary open angle glaucoma, POAG) and 11 patients with ocular hypertension and no symptoma of POAG were recruited from the Departments of Opthalmology of the University of Erlangen-Nümberg. The control groups are from the Max Delbrück Centre of Moleculare Medicine, Berlin and from the University of Erlangen- Nümberg.

The POAG was defined as an intraocular pressure >21 mm Hg.

For immunoglobulin preparation, the samples were centrifuged at 4,000 g for 30 min and stored at-20°C.

*Reagents* was purchased from Sigma-Aldrich Chemie (Deisenhofen, Germany). All other chemicals were of analytical grade.

Statistics. Student's *t* test was used to compare variables between the groups. Values of P < 0.05 are significant.

### Results

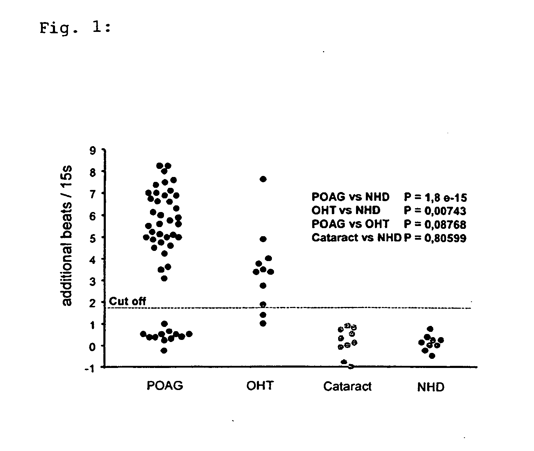

Fig. 1 shows the increase in beats per minute (bpm) of spontaneously beating neonatal rat cardiomyocytes when exposed to immunoglobulin from 48 patients with POAG. 36/48 patients (75%) stimulate the cardiomyocytes. 9/11 immunoglobulins from patients with OHT patients stimulate the cells; no stimulation was observed with immunoglobulin fractions of patients with cataracat or healthy control persons. The differences are highly significant.

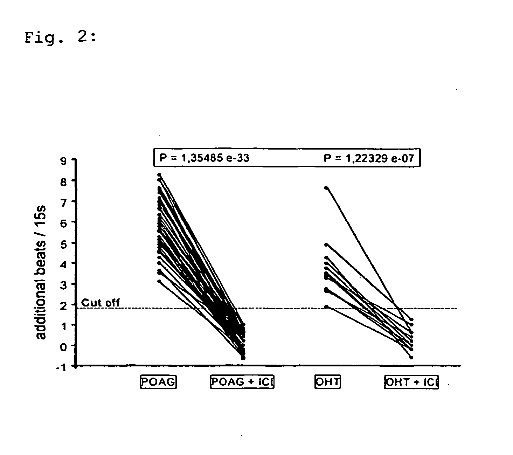

The specificity of the response to the ß2 adrenergic receptor can be demonstrated by co-incubation of the antibody stimulated cardiomyocytes with ICI 118,551, a selective ß2 receptor blocking agent (Fig.2).

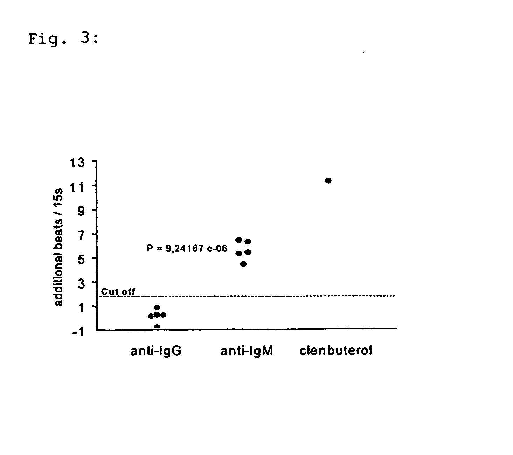

To identify the immunoglobulin classes we precipitated the immunoglobulin fraction from 5 patients with POAG with anti-human IgG and anti-human IgM antibodies. The anti-human IgG antibody abolished the effect, whereas the anti-human IgM antibody had no effect (fig.3).

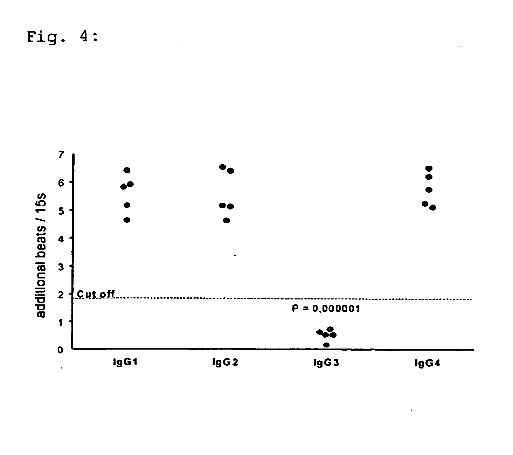

We next tested the IgG subclasses of the agonist-like ß2 adrenergic receptor Autoantibodies by preincubating of the immunoglobulin fraction from five POAG patients with monoclonal anti- human IgG₁₋₄ antibodies (Fig.4). Fig.4 shows that only the anti-IgG3 antibodies were able to neutralize the activity of the POAG antibodies. These results support the notion that antibodies of the IgG3 subclass in the immunoglobulin fraction from POAG patients were responsible for the effect.

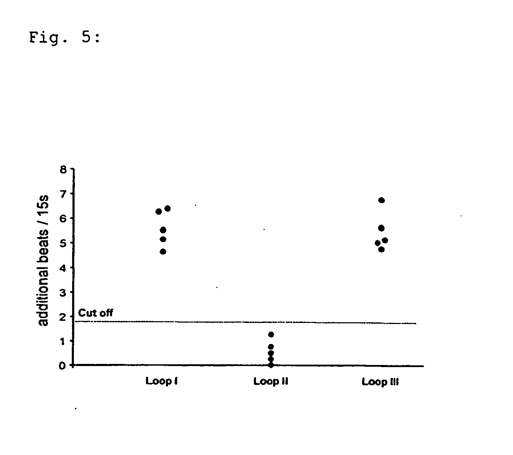

We tested immunoglobulins from 6 patients POAG who were preexposed to peptides containing the amino acid sequence of the first, second, and third extracellular ß2 receptor loops. Only peptides from the second extracellular loop blocked the effect (fig.5).

To identify the amino acid sequence within the loop II which may block the antibody activity to the ß2 receptor the immunoglobulins of 5 patients with POAG were preincubated with short, overlapping peptides corresponding to the second extracellular loop of the ß2 receptor. (fig.5). Only the amino acid sequences AINCYAN and ANETCCD of the loop II abolished the increase in the spontaneous beating rate in all 5 patients tested. These findings suggest that this sites contains the binding epitopes of the antibody (fig.5).

### Therapeutical Approach: Removal of agAAB directed against the ß2-AR

The example shows the data from a patient (E.D., 70 years old, male) with a primary open-angle glaucoma (POAG). To reduce the intraocular pressure (IOP) below 20 mmHg, the medication of 4 different drugs (eye drops) was necessary. The patient underwent filtrating surgery (trabeculectomy) on the left eye. The IOP in the fellow eye was about 30 mm Hg without antiglaucomatous therapy. AgAAb directed against the ß2-AR were demonstrated in the blood of the patients 6 months before and at the time of the start of immunoadsorption. The autoantibodies belonged to the imunoglobulin G (IgG) subclass IgG3. The targets of the autoantibodies were the epitopes of the second extracellular loop of the ß2-AR described before.

The removal of the agAAB was performed by using the immunoglobulin adsorber columns Globaffin (Fresenius AG). Two columns were used in combination with the ADAsorb plasma therapy device of *medicap clinic GmbH* (Ulrichstein, Germany) and a blood cell separator as described before (Fig.A; ref. Roenspeck W et al, Peptide Based Adsorbers for Therapeutic Immunoadsorption, in: Ther. Apheresis and Dialysis 71 (1),91-97; 2002). The immunoad-sorption was performed on five consecutive days with the two times plasma volume each day.

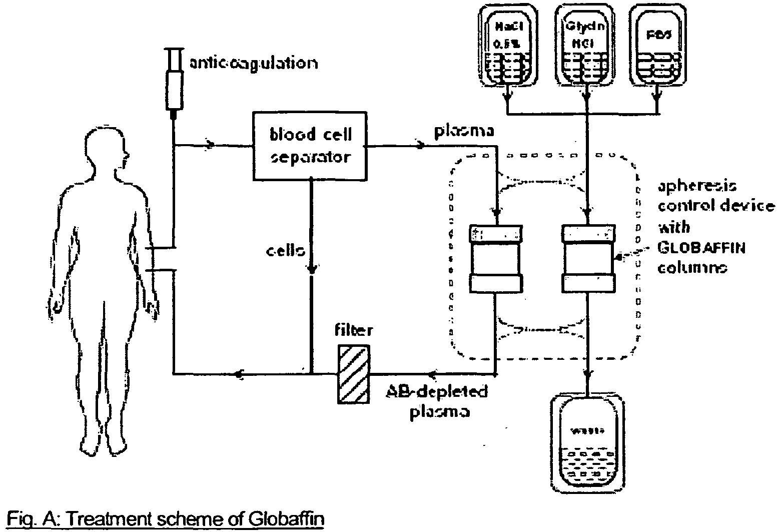
NaCl
0.[?]%
Glycin
HCl
PBS
anticoagulation
blood cell
separator
plasma
apheresis
control device
with
GLOBAFFIN
columns
cells
filter
AB-depleted
plasma
waste
Fig. A: Treatment scheme of Globaffin

### Performance of the immunoadsorption

The immunoadsorption leads to a decline of the whole plasma IgG. As to be seen in fig.B (before-nos.1, 3, 5, 7, 9 - and after- nos. 2, 4, 6, 8 ,10 - immunoadsorption) after day 5 (nr.10) about 95 % of IgG were removed from patient plasma within 5 days. The decline of IgG3 was about 80% (Fig. B). 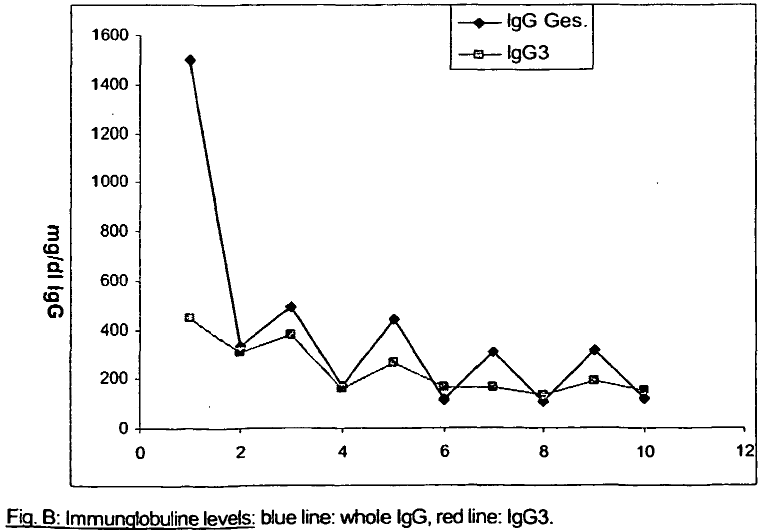 Because of the binding characteristics of the Globaffin columns to the IgG subclasses IgG3 will be adsorbed after removal of the large amounts of IgG1 and IgG2.

The effect of the immunoadsorption on the levels of the bioactive agAAB was estimated before and after immunoadsorption from collected serum samples in the bioassay. As shown in fig. C the activity of the agAAB declines parallel to the decline of IgG3.

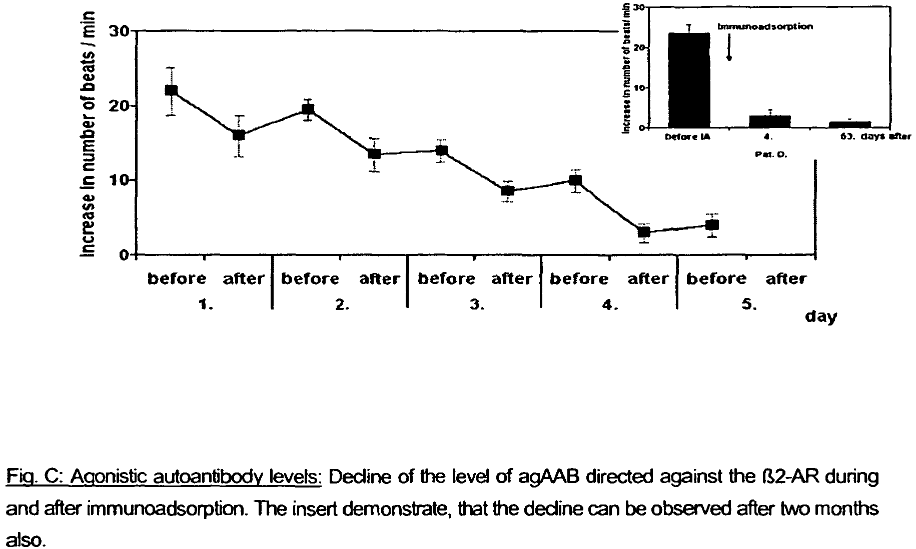

### Clinical outcome

Parallel to the immunoadsorption the intraocular pressure (IOP) was measured. Suprisingly the removal of plasma immunoglobulins leads to a decline of the IOP in both eyes. A long lasting decline of IOP could not be observed in this patient. But in contrast to the drug medication before immunoadsorption the IOP lowering effect of the drugs used was more pronounced.

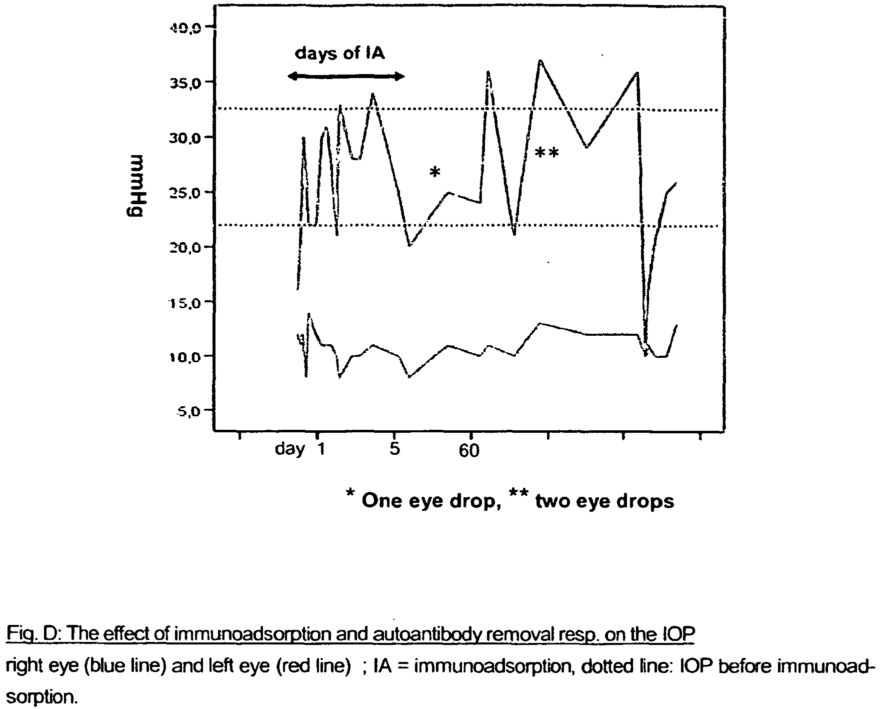

### The figures show the following:

**Fig.1****:** Antibodies from patients with POAG or OHT stimulates cultivated neonatal rat cardiomycytes. Immunoglobulin from patients wih cataract and healthy control persons are not active.
**Fig.2****:** Antibodies of patients with glaucoma (POAG) and OHT stimulate rat cardiomyocytes via ß2 adrenergic receptor. The ß2 adrenergic antagonist ICI 118,551 blocks specifically the stimulating effects of the agonistic antibodies.
Fig.3: The antibodies from patients with POAG directed against the ß2 adrenergic receptor are of the immunoglobulin G isotype. The neutralisation of IgG from the immunoglobulin fraction leads to the lost of the cell stimulation. The ß2 receptor agonist clenbuturol reflects the potential for the stimulation of the cells.
**Fig.4****:** Antibodies from 5 patients with POAG stimulating rat cardiomyocytes are of the immunoglobulin IgG₃ isotype subclass. The separation of IgG₃ subclass from the immunoglobulin fraction leads to the lost of the stimulating effect. The data are higly significant.
**Fig.5**: Antibodies from patients with POAG recognizes the loop II of the ß2 adrergic receptor. The loop II peptid blocks the antibody activity to stimulate the rat cardiomyocytes.

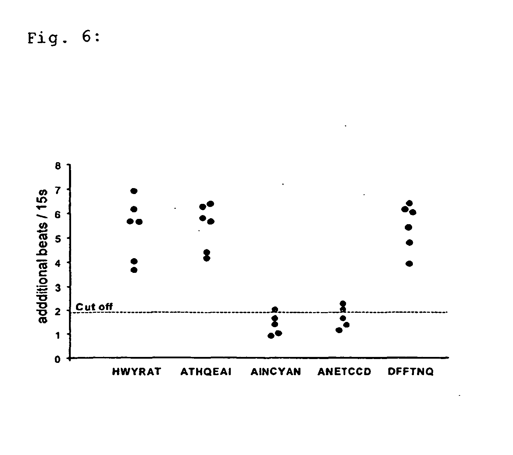

**Fig.6****:** Antibodies recognize the epitopes AINCYAN and ANETCCD of the loop II of the ß2 adrenergic receptor of the 5 overlapping peptides.

## Claims

1. A peptide, consisting of an amino acid sequence X1-X2-X3-X4-X5-X6-X7-X8-X9 wherein
X1 = amino group, amide, acetyl group, biotin group, marker, spacer, linker, GKK, SGKK or deletion,
X9 = amino group, amide, acetyl group, biotin group, marker, spacer, linker, GKK, SGKK or deletion, wherein
X2 to X8 of the amino acid sequence X1-X2-X3-X4-X5-X6-X7-X8-X9 is selected from a group that consists of
(i) a peptide consisting of the amino acid sequence GLQSWGQ, SVEATSE, VADMSVD, AQNTCCN, GNQSSSQ, SEEYAHE, AINCYAN and/orANETCCD;
(ii) a peptide consisting of an amino acid sequence having a sequence with 90% homology to an amino acid sequence in accordance with (i);
(iii) a peptide according to amino acid sequence (i) or (ii) which is modified by branch or extension with the same or another peptide according to amino acid sequence (i) or (ii) to form a homooligomeric or heterooligomeric peptide.

2. The peptide according to claim 1 for use as a medical active substance.

3. The peptide according to claims 1 or 2,
**characterized in that**
the peptide binds to antibodies of patients suffering from glaucoma.

4. An isolated nucleic acid molecule selected from the group comprising:
a) a nucleic acid molecule consisting of a nucleotide sequence which encodes at least one peptide selected from the group consisting of peptides according to claim 1;
b) a nucleic acid molecule which is complementary to a nucleotide sequence in accordance with a);
c) a nucleic acid molecule which undergoes hybridization with a nucleotide sequence according to a) or b) under stringent conditions;
d) a nucleic acid molecule consisting of a nucleotide sequence having a sequence with 90% homology to a nucleotide sequence according to a), b) or c);
e) a nucleic acid molecule which, as a consequence of the genetic code, is degenerated into a nucleotide sequence according to a) through d).

5. Solid phases for affinity chromatography or solid-phase extraction consisting of organic, inorganic, synthetic polymers or of mixed polymers, preferably cross-linked agarose, cellulose, silica gel, polyamide and polyvinyl alcohols, which are optionally chemically activated, with peptides according to at least one of claims 1 to 3, immobilized on the surface of the solid phase.

6. A pharmaceutical composition comprising a nucleic acid molecule according to claim 4, a peptide according to any of claims 1 to 3, and/or a solid phase according to claim 5, optionally together with a pharmaceutically tolerable carrier.

7. A nucleic acid molecule according to claim 4, a peptide according to any of claims 1 to 3, a solid phase according to claim 5 and/or a pharmaceutical composition according to claim 6 for the in vivo diagnosis or therapy of glaucoma.

8. Use of a nucleic acid molecule according to claim 4, a peptide according to any of claims 1 to 3, a solid phase according to claim 5 and/or a pharmaceutical composition according to claim 6, in the in vitro diagnosis of glaucoma.

9. Use of a nucleic acid molecule according to claim 4, a peptide according to any of claims 1 to 3, a solid phase according to claim 5, a pharmaceutical composition according to claim 6, in the production or screening of a drug for the treatment of diseases of an optic nerve.

10. The use according to claims 7 to 9,
**characterized in that**
the glaucoma are a primary open angle glaucoma (POAG), ocular hypertension (OHT), a normal tension glaucoma (NTG), an acute angle-closure glaucoma, a primary congenital glaucoma, pseudoexfoliant glaucoma (PEX) and/or a secondary glaucoma.

## Patentansprüche

1. Peptid, bestehend aus einer Aminosäuresequenz X1-X2-X3-X4-X5-X6-X7-X8-X9, wobei
X1 = Aminogruppe, Amid, Acetylgruppe, Biotingruppe, Marker, Spacer, Linker, GKK, SGKK oder Deletion,
X9 = Aminogruppe, Amid, Acetylgruppe, Biotingruppe, Marker, Spacer, Linker, GKK, SGKK oder Deletion, wobei
X2 bis X8 der Aminosäuresequenz X1-X2-X3-X4-X5-X6-X7-X8-X9 aus einer Gruppe ausgewählt sind, die aus Folgendem besteht:
(i) einem Peptid, das aus der Aminosäuresequenz GLQSWGQ, SVEATSE, VADMSVD, AQNTCCN, GNQSSSQ, SEEYAHE, AINCYAN und/oder ANETCCD besteht;
(ii) einem Peptid, das aus einer Aminosäuresequenz besteht, die eine 90%ige Homologie zu einer Aminosäuresequenz gemäß (i) aufweist;
(iii) einem Peptid gemäß der Aminosäuresequenz (i) oder (ii), welches durch Verzweigung oder Verlängerung mit dem gleichen oder einem anderen Peptid gemäß der Aminosäuresequenz (i) oder (ii) zur Bildung eines homooligomeren oder heterooligomeren Peptids modifiziert ist.

2. Peptid nach Anspruch 1 zur Verwendung als medizinischer Wirkstoff.

3. Peptid nach Anspruch 1 oder 2,
**dadurch gekennzeichnet, dass**
das Peptid an Antikörper von Patienten bindet, die an einem Glaukom leiden.

4. Isoliertes Nukleinsäuremolekül, ausgewählt aus der Gruppe umfassend:
a) ein Nukleinsäuremolekül, das aus einer Nukleotidsequenz besteht, welche für mindestens ein Peptid kodiert, das aus der Gruppe bestehend aus Peptiden nach Anspruch 1 ausgewählt ist;
b) ein Nukleinsäuremolekül, welches komplementär zu einer Nukleotidsequenz gemäß a) ist;
c) ein Nukleinsäuremolekül, welches eine Hybridisierung mit einer Nukleotidsequenz gemäß a) oder b) unter Stringenzbedingungen erfährt;
d) ein Nukleinsäuremolekül, das aus einer Nukleotidsequenz besteht, die eine Sequenz mit 90%iger Homologie zu einer Nukleotidsequenz gemäß a), b) oder c) aufweist;
e) ein Nukleinsäuremolekül, welches infolge des genetischen Codes zu einer Nukleotidsequenz gemäß a) bis d) degeneriert ist.

5. Festphasen für Affinitätschromatographie oder Festphasenextraktion, bestehend aus organischen, anorganischen, synthetischen Polymeren oder aus Mischpolymeren, vorzugsweise vernetzter Agarose, Cellulose, Kieselgel, Polyamid- und Polyvinylalkoholen, welche gegebenenfalls chemisch aktiviert sind, mit Peptiden nach mindestens einem der Ansprüche 1 bis 3, die auf der Oberfläche der Festphase immobilisiert sind.

6. Pharmazeutische Zusammensetzung umfassend ein Nukleinsäuremolekül nach Anspruch 4, ein Peptid nach irgendeinem der Ansprüche 1 bis 3 und/oder eine Festphase nach Anspruch 5, gegebenenfalls zusammen mit einem pharmazeutisch annehmbaren Träger.

7. Nukleinsäuremolekül nach Anspruch 4, Peptid nach irgendeinem der Ansprüche 1 bis 3, Festphase nach Anspruch 5 und/oder pharmazeutische Zusammensetzung nach Anspruch 6 für die In-vivo-Diagnose oder -Therapie eines Glaukoms.

8. Verwendung eines Nukleinsäuremoleküls nach Anspruch 4, eines Peptids nach irgendeinem der Ansprüche 1 bis 3, einer Festphase nach Anspruch 5 und/oder einer pharmazeutischen Zusammensetzung nach Anspruch 6 bei der In-vitro-Diagnose eines Glaukoms.

9. Verwendung eines Nukleinsäuremoleküls nach Anspruch 4, eines Peptids nach irgendeinem der Ansprüche 1 bis 3, einer Festphase nach Anspruch 5, einer pharmazeutischen Zusammensetzung nach Anspruch 6 bei der Herstellung oder dem Screening eines Arzneimittels zur Behandlung von Erkrankungen eines Sehnervs.

10. Verwendung nach Anspruch 7 bis 9,
**dadurch gekennzeichnet, dass**
das Glaukom ein primäres Offenwinkelglaukom (POWG), okuläre Hypertension (OHT), ein Normaldruckglaukom (NDG), ein akutes Winkelblockglaukom, ein primäres kongenitales Glaukom, ein Pseudoexfoliationsglaukom (PEG) und/oder ein sekundäres Glaukom ist.

## Revendications

1. Peptide, constitué par une séquence d'acides aminés X1-X2-X3-X4-X5-X6-X7-X8-X9, dans laquelle
X1 = un groupe amino, un amide, un groupe acétyle, un groupe biotine, un marqueur, un espaceur, un lieur, GKK, SGKK ou une délétion,
X9 = un groupe amino, un amide, un groupe acétyle, un groupe biotine, un marqueur, un espaceur, un lieur, GKK, SGKK ou une délétion, dans lequel X2 à X8 de la séquence d'acides aminés X1-X2-X3-X4-X5-X6-X7-X8-X9 est choisi dans un groupe qui est constitué par
(i) un peptide constitué de la séquence d'acides aminés GLQSWGQ, SVEATSE, VADMSVD, AQNTCCN, GNQSSSQ, SEEYAHE, AINCYAN et/ou ANETCCD ;
(ii) un peptide constitué par une séquence d'acides aminés ayant une séquence présentant 90 % d'homologie avec une séquence d'acides aminés conformément à (i) ;
(iii) un peptide selon la séquence d'acides aminés (i) ou (ii) qui est modifiée par une ramification ou une extension avec le même peptide ou un autre selon la séquence d'acides aminés (i) ou (ii) pour former un peptide homooligomère ou hétérooligomère.

2. Peptide selon la revendication 1 pour une utilisation en tant que substance active médicale.

3. Peptide selon la revendication 1 ou 2,
**caractérisé en ce que**
le peptide se lie à des anticorps de patients atteints de glaucome.

4. Molécule d'acide nucléique isolée choisie dans le groupe comprenant :
a) une molécule d'acide nucléique constituée par une séquence nucléotidique qui code pour au moins un peptide choisi dans le groupe constitué par les peptides selon la revendication 1 ;
b) une molécule d'acide nucléique qui est complémentaire d'une séquence nucléotidique conforme à a) ;
c) une molécule d'acide nucléique qui subit une hybridation avec une séquence nucléotidique selon a) ou b) dans des conditions string entes ;
d) une molécule d'acide nucléique constituée par une séquence nucléotidique ayant une séquence présentant 90 % d'homologie avec une séquence nucléotidique selon a), b) ou c) ;
e) une molécule d'acide nucléique qui, en raison du code génétique, est dégénérée en une séquence nucléotidique selon a) à d).

5. Phases solides pour une chromatographie d'affinité ou une extraction en phase solide constituées de polymères organiques, inorganiques, synthétiques ou de polymères mixtes, de préférence d'agarose réticulée, de cellulose, de gel de silice, de polyamide et de poly(alcools de vinyle), qui sont éventuellement chimiquement activées, avec des peptides selon au moins l'une des revendications 1 à 3, immobilisés sur la surface de la phase solide.

6. Composition pharmaceutique comprenant une molécule d'acide nucléique selon la revendication 4, un peptide selon l'une quelconque des revendications 1 à 3, et/ou une phase solide selon la revendication 5, éventuellement conjointement à un support pharmaceutiquement acceptable.

7. Molécule d'acide nucléique selon la revendication 4, peptide selon l'une quelconque des revendications 1 à 3, phase solide selon la revendication 5 et/ou composition pharmaceutique selon la revendication 6 pour le diagnostic in vivo ou le traitement in vivo d'un glaucome.

8. Utilisation d'une molécule d'acide nucléique selon la revendication 4, d'un peptide selon l'une quelconque des revendications 1 à 3, d'une phase solide selon la revendication 5 et/ou d'une composition pharmaceutique selon la revendication 6, dans le diagnostic in vivo du glaucome.

9. Utilisation d'une molécule d'acide nucléique selon la revendication 4, d'un peptide selon l'une quelconque des revendications 1 à 3, d'une phase solide selon la revendication 5, d'une composition pharmaceutique selon la revendication 6, dans la production ou le criblage d'un médicament destiné au traitement de maladies d'un nerf optique.

10. Utilisation selon les revendications 7 à 9,
**caractérisée en ce que**
les glaucomes sont un glaucome de l'angle ouvert primitif (POAG), une hypertension oculaire (OHT), un glaucome à tension normale (NTG), un glaucome aigu par fermeture de l'angle, un glaucome primitif congénital, un glaucome pseudo-exfoliatif (PEX) et/ou un glaucome secondaire.
